# FASCICULE DE BREVET EUROPEEN

(11) **EP 0 742 013 B1**
(45) Date de publication et mention de la délivrance du brevet: **01.09.1999**
(21) Numéro de dépôt: 96400995.5
(22) Date de dépôt: 09.05.1996
(51) Int. Cl.: A61K 47/10, A61K 47/26, A61K 47/18, A61K 9/14, A61K 38/44

(54) **Composition liquide stable contenant de l'urate oxydase et composition lyophilisée pour sa préparation**
Stabile flüssige Zusammensetzung enthaltend Urateoxidase und lyophilizierte Zusammensetzung für seine Herstellung
Stable liquid composition containing urate oxidase and lyophilized composition for its preparation

(30) Priorité: 11.05.1995 FR 9505606
(43) Date de publication de la demande: 13.11.1996
(73) Titulaire: SANOFI, 75008 Paris (FR)
(72) Inventeur: Aleman, Claude, 34660 Cournonterral (FR); Bayol, Alain, 31170 Tournefeuille (FR); Breul, Thierry, 34080 Montpellier (FR); Dupin, Patrice, 31520 Ramonville Saint Agne (FR)
(74) Mandataire: Le Guen, Gérard

(56) Documents cités:
- EP-A- 0 069 379
- EP-A- 0 291 060
- US-A- 3 928 137
- CHEMICAL ABSTRACTS, vol. 104, no. 15, 14 Avril 1986 Columbus, Ohio, US; abstract no. 125711, H. YUZO: "Stable uricase preparation" XP002011166
- AMERICAN PHARMACEUTICAL ASSOCIATION AND PHARMACEUTICAL SOCIETY OF GREAT BRITAIN: "Handbook of Pharmaceutical Excipients" 1986 , AMERICAN PHARMACEUTICAL ASSOC. , WASHINGTON XP002011216 153140 * page 207 - page 208 *

## Description

La présente invention concerne une formulation liquide pharmaceutiquement acceptable contenant de l'urate oxydase,se présentant sous forme d'une solution stable à 25°C et limpide après agitation.

Cette solution peut être obtenue également par dissolution d'un lyophilisat contenant de l'urate oxydase dans un solvant.

L'urate oxydase (urate oxygen oxidoreductase EC 1.7.3.3 Uox), une enzyme protéique obtenue à partir d'*Aspergillus flavus* qui oxyde l'acide urique en allantoïne, est utilisée pour la prévention ou le traitement de l'hyperuricémie lors des chimiothérapies, car l'allantoïne est dix fois plus soluble que l'acide urique, et facilement éliminée par voie rénale (Laboureur P. et al., Bull. Soc. Chim. Biol., 1968, 50, 811-825 ; Kissel P. et al., The Lancet, 1975, J25, 229).

L'urate oxydase est une enzyme tétramère composée de quatre unités identiques d'un poids moléculaire de 34152. Chaque unité monomère, formée d'une seule chaine polypeptidique de 301 acides aminés est acétylée à l'extrémité N-terminale et ne présente pas de ponts disulfures. Le pH optimal de stabilité de l'activité enzymatique de l'urate oxydase en solution est pH = 8 (Bayol, A. et al., accepté pour publication Biophys. Chem., 1995 (54), 229-35).

L'ADNc codant pour cette protéine a été cloné et exprimé dans *E. coli* (Legoux, R., et al. J. Biol. Chem., 1992, 267, (12), 8565-8570); dans *A. flavus* (Chevalet, L., et al., Curr. Genet., 1992, 21, 447-453) ; et dans *S. cerevisiae* (Leplatois, P., et al., Gene., 1992, 122, 139-145). Les meilleurs rendements obtenus à partir de *S. cerevisiae* ont favorisé le choix de cette levure pour la production d'urate oxydase recombinante (re-Uox). U'enzyme recombinante accumulée dans l'espace intracellulaire sous une forme soluble et active est extraite puis purifiée jusqu'à l'obtention d'une qualité pharmaceutique.

Afin d'obtenir une formulation administrable à l'homme, l'urate oxydase doit être préparée sous forme d'une composition pharmaceutique. De telles compositions doivent conserver l'activité enzymatique de l'urate oxydase dans le temps. On utilise généralement une forme lyophilisée pour conserver l'intégrité biochimique et l'activité biologique des enzymes dans les conditions de conservation les plus variées. Il est bien connu que les préparations lyophilisées conservent mieux ces propriétés que les préparations liquides correspondantes.

Les préparations lyophilisées doivent être diluées dans un solvant pharmaceutiquement acceptable avant administration à l'homme par voie parentérale. Une composition liquide de l'enzyme serait néanmoins préférable car elle serait plus rapidement administrable, à condition toutefois d'être stable physiquement et chimiquement.

La formulation pharmaceutique connue de l'urate oxydase (Uricozyme®) est une préparation injectable lyophilisée nécessitant un solvant de reconstitution. La composition de la forme lyophilisée de l'Uricozyme® est la suivante :

| | |
|---|---|
| Urate oxydase | 1000 Unités |
| Aza-8-dioxo-2,6 purine monohydrate | |
| Sodium carbonate neutre anhydre | |
| Tétracémate disodique dihydraté | 0,37 mg |
| Lactose | |

La composition du solvant de reconstitution est la suivante :

| | |
|---|---|
| Potassium phosphate monoacide | |
| Potassium phosphate diacide | |
| Glucose | |
| Eau pour préparations injectables | q.s.p. 1 ml |

(Dictionnaire VIDAL Paris 61ème édition p.1552, 1985.)

La solution d'urate oxydase obtenue après dissolution du lyophilisat dans le solvant de reconstitution voit sa turbidité augmenter avec le temps de conservation à 25°C jusqu'à l'apparition d'un précipité protéïque flamenteux. La formation de ce précipité protéique en solution est accélérée par le temps et la force d'agitation de la solution. La solution obtenue est physiquement instable.

Les propriétés solubilisantes des molécules tensioactives non ioniques sont bien connues de l'homme de l'art, mais les interactions entre protéines et surfactifs non ioniques sont plus spécifiques et décrites dans la littérature.

Le brevet EP 211 601 décrit l'utilisation de certains copolymères séquencés, qui sont des molécules tensioactives non ioniques, dans la stabilisation de formulations injectables d'hormone de croissance. L'hormone de croissance est stabilisée dans une matrice gélifiée formée par un copolymère séquencé contenant des unités polyoxyéthylénées et polyoxypropylénées de poids moléculaire moyen compris entre 1100 et 40000.

Le brevet américain U.S. 4,783,441 décrit une méthode de prévention de la dénaturation des protéines comme l'insuline en solution aqueuse, par addition d'une quantité pouvant aller jusqu'à 500 ppm de molécules tensioactives composées de groupements légèrement hydrophiles et légèrement hydrophobes altemés, en solutions de pH compris entre 6,8 et 8.

Le brevet U.S. 5,096,885 décrit la composition d'une solution à lyophiliser contenant l'hormone de croissance humaine, de la glycine, du mannitol, un surfactif non ionique et un tampon.

US 3 928 137 décrit une composition d'urate oxydase tamponnée et stabilisée par un tensioactif qui est un copolymère azoté de polyoxypropylène et de polyoxyéthylène. Cette composition est une composition solide stable physiquement. Néanmoins, ce document ne donne aucune indication sur le comportement et notamment sur la stabilité physique des compositions aqueuses résultant de la dissolution dans une solution aqueuse d'une telle composition solide.

JP 60 224 499 concerne une préparation liquide stable d'urate oxydase comprenant un ou plusieurs tensioactifs non-ioniques, de l'albumine de sérum et éventuellement un ou plusieurs aminoacides basiques. D'après ce document, l'incorporation d'albumine de sérum en combinaison avec un ou plusieurs tensioactifs est essentielle pour assurer la stabilité physique des compositions liquides, et ceci même après lyophilisation et redissolution dans une solution aqueuse.

L'objet de la présente invention est une composition liquide pharmaceutiquement acceptable d'une solution d'urate oxydase contenant entre 0,1 mg/ml et 10 mg/ml d'un copolymère séquencé d'oxyde d'éthylène et d'oxyde de propylène, le Poloxamer 188, en milieu aqueux tamponné. Cette composition est limpide et stable physiquement et chimiquement après une vigoureuse agitation ou après conservation, pendant au moins 48 heures et jusqu'à un an à 25°C.

Les avantages offerts par cette forme sont une plus grande simplicité d'utilisation en clinique, liée à la possibilité d'être administrée directement et d'être stockée sous sa forme directement administrable plus longtemps.

Un autre avantage réside en l'absence de consignes particulières de précautions de manutention habituellement nécessaires pour éviter la précipitation de filaments protéïques dans la solution à injecter, car la composition selon l'invention a la propriété d'éviter la précipitation de filaments protéiques au sein de la solution.

La composition liquide aqueuse de l'invention est limpide, stable, stérile et pharmaceutiquement acceptable, et peut être injectée à l'homme ou à l'animal par voie sous-cutanée, intraveineuse ou intramusculaire.

Dans la présente invention, il est bien entendu que le terme urate oxydase désigne la protéine obtenue par fermentation d'une souche naturelle ou mutée par génie génétique. L'enzyme est donc produite par extraction de source naturelle, ou à partir de cultures cellulaires. L'urate oxydase utilisée dans les formulations de la présente invention peut être obtenue par exemple selon les documents US 3,810,820, DD 284 689, DD 296 804, DD300 781, DE 2 164 018, DE 1 517 742, FR 2 664 286, GB 2 221 910, JP 76-007749, JP-75-030137, JP-84-023987,JP73 018473, JP-47 029575, SU 565 935, US 4,062,731, EP 545 688 ou selon EP 435 776.

Le terme quantité efficace pharmaceutiquement désigne toute quantité d'urate oxydase qui produit un effet thérapeutique.

Les solutions corrrespondantes à l'invention contiennent une quantité efficace pharmaceutiquement d'urate oxydase. Préférentiellement ces solutions contiennent entre 0,1 mg/ml et 50 mg/ml d'urate oxydase selon le dosage désiré. Le domaine de concentration en urate oxydase n'est pas critique pour l'invention et peut varier selon les préparations.

Les solutions correspondantes à l'invention contiennent un surfactif non ionique de type copolymère séquencé de polyoxyéthylène et de polyoxypropylène dénommé Poloxamer 188 et répondant à la formule HO(CH₂CH₂O)₇₅(CH(CH₃)CH₂O)₃₀(CH₂CH₂O)₇₅H. Ce produit est commercialisé par la société I.C.I. sous le nom de Synperonic F 68® et par la société B.A.S.F. sous le nom de Pluronic F 68®. Les quantités nécessaires de Poloxamer 188 pour obtenir des solutions physiquement stables d'urate oxydase sont comprises entre 0,1 mg/ml et 10 mg/ml, de préférence entre 0,5 mg/ml et 5 mg/ml.

Le pH de la solution est préférentiellement compris entre 7,5 et 8,5. Lorsque le pH = 8 l'urate oxydase est particulièrement stable chimiquement en solution.

De manière générale, on préfère utiliser un tampon phosphate de sodium pour tamponner le milieu. La concentration du tampon est avantageusement de 5mM à 100 mM.

Le tampon préférentiellement utilisé est un tampon phosphate de sodium à pH = 8 à une concentration comprise entre 5 mM et 100 mM.

La composition liquide selon l'invention est de préférence isotonique et comprend avantageusement des excipients nécessaires à l'obtention d'une solution isotonique, comme du mannitol ou de l'alanine.

La solution peut contenir en particulier du mannitol, de préférence à une concentration comprise entre 1 mg/ml et 50 mg/ml.

La solution peut contenir en particulier de l'alanine, de préférence à une concentration comprise entre 1 mg/ml et 50 mg/ml.

La solution contient préférentiellement du mannitol et de l'alanine en toute proportion qui conserve l'isotonicité de la solution, de préférence de 1 à 50 mg/ml de mannitol et de 1 à 50 mg/ml d'alanine.

Avantageusement, la solution contient 10,6 mg/ml de mannitol et 15,9 mg/ml d'alanine.

La composition liquide selon l'invention comprend avantageusement des conservateurs nécessaires à la conservation bactériologique de la solution, comme notamment l'alcool benzylique, le phénol, le méta-crésol, le méthyl paraben, le propyl paraben, un chlorure de benzalkonium ou chlorure de benzethonium.

La solution peut être obtenue directement par dissolution des constituants dans de l'eau ; ou après reconstitution d'un lyophilisat contenant l'urate oxydase, par un solvant aqueux contenant le Poloxamer 188 ; ou après reconstitution d'un lyophilisat contenant l'urate oxydase et le Poloxamer 188, par un solvant aqueux.

A cet égard, l'invention a également pour objet une composition lyophilisée à dissoudre dans un solvant aqueux, et contenant de l'urate oxydase et du Poloxamer 188, le rapport pondéral du Poloxamer 188 à l'urate oxydase étant de 0,01 à 50.

La composition lyophilisée peut avantageusement comprendre en outre un tampon, comme indiqué précédemment pour la solution.

Elle peut également comprendre avantageusement des excipients assurant l'isotonie de la solution aqueuse obtenue par dissolution du lyophilisat dans un solvant aqueux, comme par exemple de l'alanine ou du mannitol, en des quantités variables en fonction du volume de solvant de reconstitution à utiliser.

Les exemples suivants illustrent l'invention

### EXEMPLE 1

Solution d'urate oxydase préparée par dissolution des constituants dans de l'eau

| | |
|---|---|
| Urate oxydase | 1,5 mg |
| Mannitol | 10,6 mg |
| L-Alanine | 15,9 mg |
| Phosphate de sodium dibasique dodécahydraté | 14,32 mg |
| Poloxamer 188 | 1 mg |
| Eau pour préparations injectables q.s.p | 1 ml |

### EXEMPLE 2

Solution d'urate oxydase préparée à partir d'un lyophilisat d'urate oxydase reconstitué par un solvant de reprise aqueux contenant du Poloxamer 188

### Composition du lyophilisat :

| | |
|---|---|
| Urate oxydase | 1,5 mg |
| Mannitol | 10,6 mg |
| L-Alanine | 15,9 mg |
| Phosphate de sodium dibasique dodécahydraté | 14,32 mg |

### Composition du solvant de reprise :

| | |
|---|---|
| Poloxamer 188 | 1 mg |
| Eau pour préparations injectables q.s.p | 1 ml |

### EXEMPLE 3

Solution d'urate oxydase préparée à partir d'un lyophilisat d'urate oxydase contenant du Poloxamer 188 reconstitué par de l'eau.

### Composition du lyophilisat :

| | |
|---|---|
| Urate oxydase | 1,5 mg |
| Mannitol | 10,6 mg |
| L-Alanine | 15,9 mg |
| Phosphate de sodium dibasique dodécahydraté | 14,32 mg |
| Poloxamer 188 | 1 mg |

### Composition du solvant de reprise :

| | |
|---|---|
| Eau pour préparations injectables q.s.p | 1 ml |

Les avantages des compositions de l'invention vont maintenant être mis en évidence par la comparaison des propriétés de différentes compositions à base d'urate oxydase. résumée dans le tableau I et sur les figures annexées.

### PREPARATION DES COMPOSITIONS

Les solutions d'urate oxydase correspondantes à l'invention peuvent être obtenues de différentes façons, par exemple :
- par mélange d'une solution aqueuse concentrée d'urate oxydase contenant un tampon phosphate de sodium à pH =8, avec une solution aqueuse contenant du Poloxamer 188 et éventuellement des excipients qui assurent l'isotonie de la solution mélangée, et éventuellement des conservateurs.
- par dissolution d'un lyophilisat contenant l'urate oxydase, un tampon phosphate de sodium à pH = 8, des excipients de ballast qui assurent l'isotonie comme l'alanine et le mannitol ; dans un solvant aqueux contenant du Poloxamer 188 et éventuellement des conservateurs.
- par dissolution d'un lyophilisat contenant l'urate oxydase, un tampon phosphate de sodium pH = 8, des excipients de ballast comme l'alanine et le mannitol, et du Poloxamer 188 ; dans un solvant aqueux contenant éventuellement des conservateurs.

Les solutions obtenues par ces trois procédés peuvent être filtrées stérilement.

Elles conservent leur stabilité physique ainsi que la stabilité chimique et l'activité enzymatique de l'urate oxydase pendant 1 mois à 25°C.

Différentes méthodes analytiques ont été utilisées pour mesurer différents paramètres, tels que la turbidité ou le dosage de l'activité enzymatique.

### 1. Mesure de la turbidité :

La turbidité des solutions d'urate oxydase est déterminée par un turbidimètre Ratio Hach. Les résultats de turbidité sont exprimés en Unités Néphélométriques de turbidité (NTU) définies par : Standard methods for the examination of water and wastewater de l'American Public Health Association. La mesure de turbidité indique le degré d'agrégation de l'urate oxydase en solution.

### 2. Dosage de l'activité enzymatique de l'urate oxydase :

L'activité enzymatique de l'urate oxydase est déterminée par spectrophotométrie dans une cuve thermostatée à 30°C en suivant la disparition de l'acide urique à 292 nm (Legoux, R. et al., J. Biol. Chem., 1992, 267 (12) 8565-8570).

Des essais de stabilité du soluté reconstitué d'après l'exemple 2 ont été effectués à 25°C. Le Tableau I ci-après montre la stabilité de solutés reconstitués par dissolution avec de l'eau et avec des solvants contenant respectivement 1 mg de Polysorbate 80 (Tween 80)® ou 1 mg de Poloxamer 188 selon l'invention, avec une quantité suffisante d'eau pour produire un volume de soluté de 1 ml.

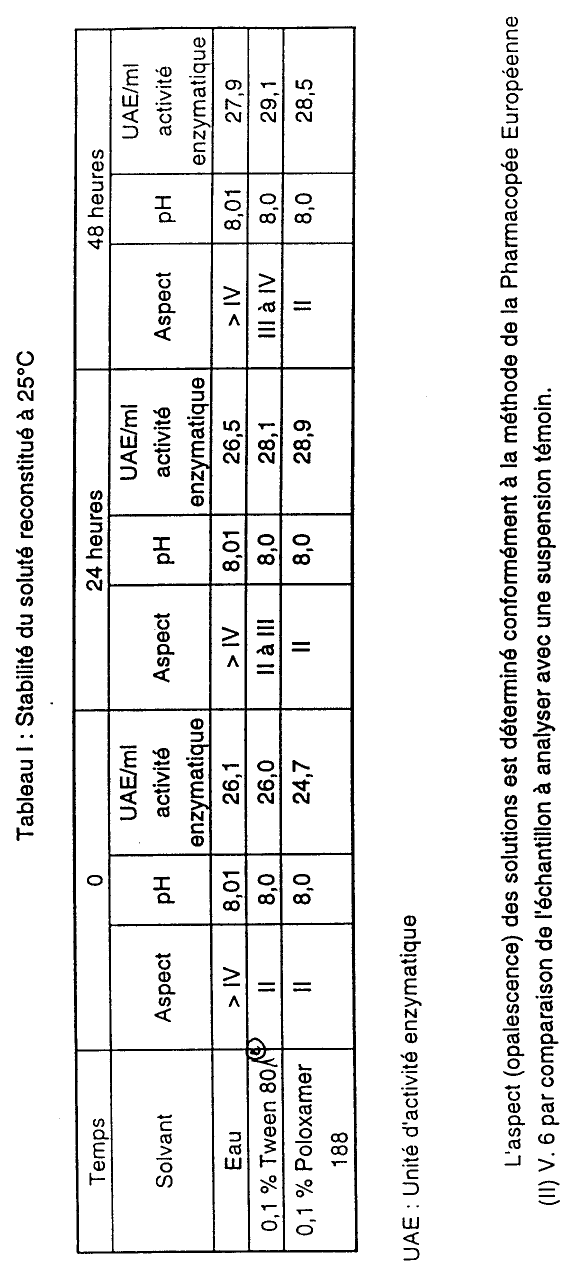

### DESCRIPTION DES FIGURES

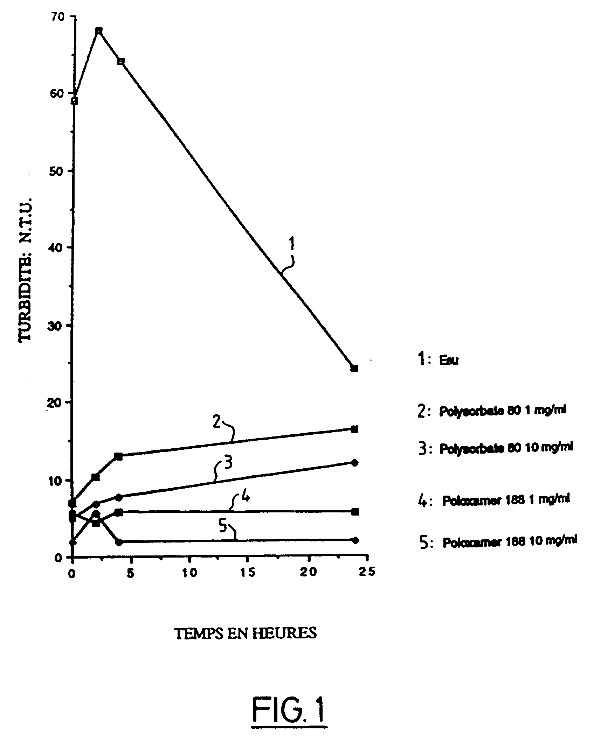

La FIGURE n° 1 indique l'évolution pendant 24 heures de la turbidité en NTU, après agitation pendant 1 minute au vortex, de cinq solutions d'urate oxydase correspondant ou non à l'invention, en fonction de leurs teneurs en tensioactifs non ioniques.

Composition n° 1 : 15 mg d'urate oxydase, 106 mg de mannitol, 159 mg de L-alanine, 143,2 mg de phosphate disodique dodécahydraté et 10 ml d'eau pour préparations injectables.

Composition n° 2 : 15 mg d'urate oxydase, 106 mg de mannitol, 159 mg de L-alanine, 143,2 mg de phosphate disodique dodécahydraté , 10 mg de Polysorbate 80 et 10 ml d'eau pour préparations injectables.

Composition n°4 (selon l'invention) : 15 mg d'urate oxydase, 106 mg de mannitol, 159 mg de L-alanine, 143,2 mg de phosphate disodique dodécahydraté, 10 mg de Poloxamer 188 et 10 ml d'eau pour préparations injectables.

Composition n°3 : 15 mg d'urate oxydase, 106 mg de mannitol, 159 mg de L-alanine, 143,2 mg de phosphate disodique dodécahydraté , 100 mg de Polysorbate 80 et 10 ml d'eau pour préparations injectables.

Composition n° 5 (selon l'invention) : 15 mg d'urate oxydase, 106 mg de mannitol, 159 mg de L-alanine, 143,2 mg de phosphate disodique dodécahydraté, 100 mg de Poloxamer 188 et 10 ml d'eau pour préparations injectables.

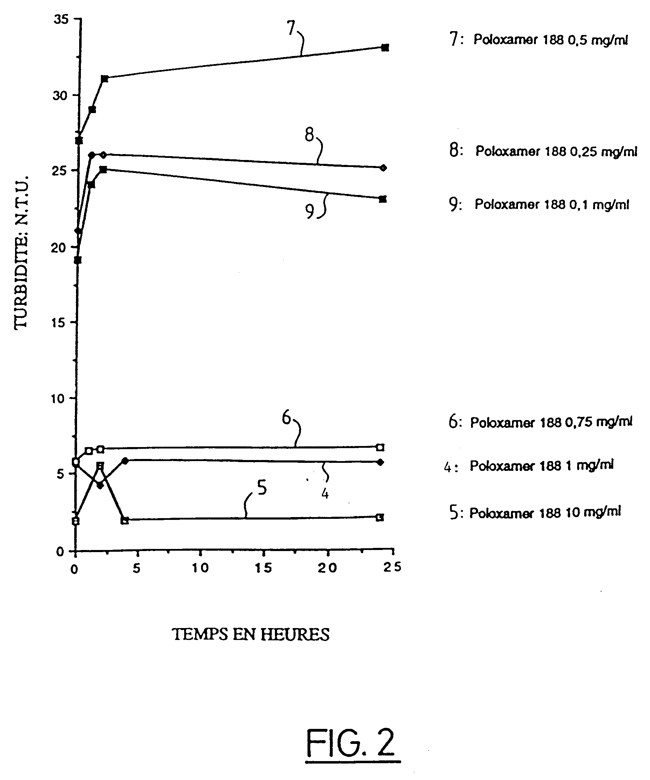

La FIGURE n° 2 indique l'évolution pendant 24 heures de la turbidité en NTU, après agitation pendant 1 minute au vortex, de six solutions d'urate oxydase contenant du Poloxamer 188 selon l'invention, en fonction de leur teneur en Poloxamer 188.

Composition n° 5 : 15 mg d'urate oxydase, 106 mg de mannitol. 159 mg de L-alanine, 143,2 mg de phosphate disodique dodécahydraté. 100 mg de Poloxamer 188 et 10 ml d'eau pour préparations injectables.

Composition n° 4 : 15 mg d'urate oxydase, 106 mg de mannitol, 159 mg de L-alanine, 143,2 mg de phosphate disodique dodécahydraté , 10 mg de Poloxamer 188 et 10 ml d'eau pour préparations injectables.

Composition n° 6 : 15 mg d'urate oxydase, 106 mg de mannitol, 159 mg de L-alanine, 143,2 mg de phosphate disodique dodécahydraté , 7,5 mg de Poloxamer 188 et 10 ml d'eau pour préparations injectables.

Composition n° 7 : 15 mg d'urate oxydase, 106 mg de mannitol, 159 mg de L-alanine, 143,2 mg de phosphate disodique dodécahydraté , 5 mg de Poloxamer 188 et 10 ml d'eau pour préparations injectables.

Composition n° 8 : 15 mg d'urate oxydase, 106 mg de mannitol, 159 mg de L-alanine, 143,2 mg de phosphate disodique dodécahydraté , 2,5 mg de Poloxamer 188 et 10 ml d'eau pour préparations injectables.

Composition n° 9 : 15 mg d'urate oxydase, 106 mg de mannitol, 159 mg de L-alanine, 143,2 mg de phosphate disodique dodécahydraté , 1 mg de Poloxamer 188 et 10 ml d'eau pour préparations injectables.

## Revendications

1. Composition liquide pharmaceutiquement acceptable, stable physique ment, contenant de l'urate oxydase, et de 0,1 mg/ml à 10 mg/ml de Poloxamer 188, en milieu aqueux tamponné.

2. Composition selon la revendication 1, contenant entre 0,5 mg/ml et 5 mg/ml de Poloxamer 188.

3. Composition selon la revendication 1, contenant en outre de l'alanine.

4. Composition selon la revendication 3, dans laquelle la quantité d'alanine est comprise entre 1 mg/ml et 50 mg/ml.

5. Composition selon la revendication 1, contenant en outre du mannitol.

6. Composition selon la revendication 5, dans laquelle la quantité de mannitol est comprise entre 1 mg/ml et 50 mg/ml.

7. Composition selon la revendication 1, contenant en outre de l'alanine et du mannitol.

8. Composition selon la revendication 7, contenant de 1 à 50 mg/ml d'alanine et de 1 à 50 mg/ml de mannitol.

9. Composition selon la revendication 8, contenant 15,9 mg/ml d'alanine et 10,6 mg/ml de mannitol.

10. Composition selon la revendication 1, isotonique.

11. Composition selon la revendication 1, contenant un tampon phosphate de sodium.

12. Composition selon la revendication 1, dans laquelle la concentration du tampon est comprise entre 5 mM et 100 mM.

13. Composition selon la revendication 1, de pH compris entre 7,5 et 8,5.

14. Composition selon la revendication 1, contenant un ou plusieurs conservateurs choisis parmi le phénol, l'alcool benzylique, le méta-crésol, le méthyl paraben, le propyl paraben, un chlorure de benzalkonium ou chlorure de benzethonium.

15. Composition selon la revendication 1, obtenue par dissolution d'un lyophilisat dans un solvant aqueux.

16. Composition selon la revendication 1,stérile et injectable à l'homme ou à l'animal par voie sous-cutanée, intraveineuse, ou intramusculaire.

17. Composition injectable selon la revendication 15 et la revendication 16, dans laquelle la composition du lyophilisat est :
| | |
|---|---|
| Urate oxydase | 1,5 mg |
| Mannitol | 10,6 mg |
| L-Alanine | 15,9 mg |
| Phosphate disodique dodécahydraté | 14,32 mg |
et la composition du solvant aqueux de reprise est :
| | |
|---|---|
| Poloxamer 188 | 1 mg |
| Eau pour préparations injectables q.s.p | 1 ml |

18. Composition lyophilisée à dissoudre dans un solvant aqueux, contenant de l'urate oxydase et du Poloxamer 188, le rapport pondéral du Poloxamer 188 à l'urate oxydase étant de 0,01 à 50.

19. Composition lyophilisée selon la revendication 18, contenant en outre un tampon.

20. Composition lyophilisée selon la revendication 18,contenant en outre des excipients assurant l'isotonie de la solution aqueuse obtenue par dissolution du lyophilisat dans un solvant aqueux.

## Claims

1. Physically stable, pharmaceutically acceptable liquid composition containing oxidase urate, and 0.1 mg/ml to 10 mg/ml of Poloxamer 188 in a buffered aqueous medium,

2. Composition according to claim 1, containing between 0.5 mg/ml and 5 mg/ml of Poloxamer 188.

3. Composition according to claim 1, further containing alanine,

4. Composition according to claim 3, wherein the quantity of alanine is between 1 mg/ml and 50 mg/ml.

5. Composition according to claim 1, further containing mannitol.

6. Composition according to claim 5, wherein the quantity of mannitol is between 1 mg/ml and 50 mg/ml.

7. Composition according to claim 1, further containing alanine and mannitol.

8. Composition according to claim 7, containing from 1 to 50 mg/ml of alanine and from 1 to 50 mg/ml of mannitol.

9. Composition according to claim 8, containing 15.9 mg/ml of alanine and 10.6 mg/ml of mannitol.

10. Composition according to claim 1 which is isotonic.

11. Composition according to claim 1 containing a sodium phosphate buffer.

12. Composition according to claim 1, wherein the concentration of the buffer is between 5 mM and 100 mM.

13. Composition according to claim 1 with a pH of between 7.5 and 8.5.

14. Composition according to claim 1, containing one or more preservatives selected from among phenol, benzyl alcohol, meta-cresol, methyl paraben, propyl paraben, a benzalkonium chloride or benzethonium chloride.

15. Composition according to claim 1, obtained by dissolving a lyophilisate in an aqueous solvent.

16. Composition according to claim 1 which is sterile and injectable into humans or animals by subcutaneous, intravenous or intramuscular route.

17. Injectable composition according to claim 15 and claim 16, wherein the composition of the lyophilisate is:
| | |
|---|---|
| oxidase urate | 1.5 mg |
| mannitol | 10.6 mg |
| L-alanine | 15.9 mg |
| disodium phosphate dodecahydrate | 14.32 mg |
and the composition of the aqueous solvent for reconstitution is:
| | |
|---|---|
| Poloxamer 188 | 1 mg |
| water for injections q.s. ad | 1 ml |

18. Lyophilised composition for dissolving in an aqueous solvent, containing oxidase urate and Poloxamer 188, the ratio by weight of Poloxamer 188 to the oxidase urate being from 0.01 to 50.

19. Lyophilised composition according to claim 18, further containing a buffer.

20. Lyophilised composition according to claim 18, further containing excipients for ensuring that the aqueous solution obtained by dissolving the lyophilisate in an aqueous solvent is isotonic.

## Patentansprüche

1. Flüssige, pharmazeutisch annehmbare, physikalisch stabile Zubereitung enthaltend Uratoxidase und 0,1 mg/ml bis 10 mg/ml Poloxamer 188 in wäßrigem gepuffertem Medium.

2. Zubereitung nach Anspruch 1, enthaltend zwischen 0.5 mg/ml und 5 mg/ml Poloxamer 188.

3. Zubereitung nach Anspruch 1, enthaltend zusätzlich Alanin.

4. Zubereitung nach Anspruch 3, worin die Menge des Alanins zwischen 1 mg/ml und 50 mg/ml liegt.

5. Zubereitung nach Anspruch 1, enthaltend zusätzlich Mannit.

6. Zubereitung nach Anspruch 5, worin die Menge des Mannits zwischen 1 mg/ml und 50 mg/ml liegt.

7. Zubereitung nach Anspruch 1, enthaltend zusätzlich Alanin und Mannit.

8. Zubereitung nach Anspruch 7, enthaltend 1 bis 50 mg/ml Alanin und 1 bis 50 mg/ml Mannit.

9. Zubereitung nach Anspruch 8, enthaltend 15,9 mg/ml Alanin und 10,6 mg/ ml Mannit.

10. Isotonische Zubereitung nach Anspruch 1.

11. Zubereitung nach Anspruch 1, enthaltend einen Natriumphosphatpuffer.

12. Zubereitung nach Anspruch 1, worin die Konzentration des Puffers zwischen 5 mM und 100 mM liegt.

13. Zubereitung nach Anspruch 1 mit einem pH-Wert zwischen 7,5 und 8,5.

14. Zubereitung nach Anspruch 1, enthaltend ein oder mehrere Konservierungsmittel ausgewählt aus Phenol, Benzylalkohol, m-Cresol, Methylparaben, Propylparaben, ein Benzalkoniumchlorid oder Benzethoniumchlorid.

15. Zubereitung nach Anspruch 1, erhalten durch Auflösen eines gefriergetrockneten Materials in einem wäßrigen Lösungsmittel.

16. Zubereitung nach Anspruch 1, die steril und auf subkutanem, intravenösem oder intramuskulärem Wege Menschen oder Tieren injiziert werden kann.

17. Injizierbare Zubereitung nach Anspruch 15 und Anspruch 16, worin die Zusammensetzung des gefriergetrockneten Materials die folgende ist:
| | |
|---|---|
| Uratoxidase | 1,5 mg |
| Mannit | 10,6 mg |
| L-Alanin | 15,9 mg |
| Dinatriumphosphat-Dodecahydrat | 14,32 mg |
und die Zusammensetzung des wäßrigen Lösungsmittels zur Herstellung die folgende ist:
| | |
|---|---|
| Poloxamer 188 | 1 mg |
| Wasser für Injektionszwecke | ad 1 ml. |

18. Gefriergetrocknete Zubereitung zur Auflösung in einem wäßrigen Lösungsmittel, enthaltend Uratoxidase und Poloxamer 188, wobei das Gewichtsverhältnis von Poloxamer 188 zu Uratoxidase 0,01 bis 50 beträgt.

19. Gefriergetrocknete Zubereitung nach Anspruch 18, enthaltend zusätzlich einen Puffer.

20. Gefriergetrocknete Zubereitung nach Anspruch 18, enthaltend zusätzlich Hilfsstoffe, welche die Isotonie der durch Auflösen des gefriergetrockneten Materials in einem wäßrigen Lösungsmittel gebildeten wäßrigen Lösung sicherstellt.
